# EUROPÄISCHE PATENTSCHRIFT

(11) **EP 1 670 388 B1**
(45) Veröffentlichungstag und Bekanntmachung des Hinweises auf die Patenterteilung: **30.07.2008**
(21) Anmeldenummer: 04765647.5
(22) Anmeldetag: 28.09.2004
(51) Int. Cl.: A61F 2/06

(54) **EINFÜHRSYSTEM MIT EINEM SELBSTEXPANDIERENDEN STENT**
INSERT SYSTEM COMPRISING AN AUTOMATICALLY EXPANDING STENT
SYSTEME D'INTRODUCTION COMPORTANT UN STENT AUTOEXPANSIBLE

(30) Priorität: 30.09.2003 DE 10346200
(43) Veröffentlichungstag der Anmeldung: 21.06.2006
(73) Patentinhaber: JOTEC GmbH, 72379 Hechingen (DE)
(72) Erfinder: KAUFMANN, Ralf, 72414 Rangendingen (DE); MÜLLER, Hardy, 72406 Bisingen (DE); LESMEISTER, Rainer, 72770 Reutlingen (DE); BRAUN, Michael, 71522 Backnang (DE); GEIS, John, 26160 Bad Zwischenahn (DE)
(74) Vertreter: Laufer, Gabriele
(86) Internationale Anmeldenummer: PCT/EP2004/010829
(87) Internationale Veröffentlichungsnummer: WO 2005/032423

(56) Entgegenhaltungen:
- EP-A- 0 941 716
- WO-A-01/34240
- WO-A-02/22053
- US-A- 5 976 153
- US-A- 6 042 588

## Beschreibung

Die vorliegende Erfindung betrifft ein Einführsystem mit einem selbstexpandierenden Stent zur Implantation in ein Blutgefäß, insbesondere im Bereich des Aortenbogens, wobei der Stent einen hohlzylindrischen Körper aufweist, der zur Implantation radial komprimiert ist, und mit einer den Stent umgebenden und diesen radial zusammendrückenden Rückzughülle zur Positionierung und Freisetzung des Stents in dem Blutgefäß.

Derartige Einführsysteme und Stents sind aus dem Stand der Technik allgemein bekannt.

Mit derartigen Einführsystemen werden auch als endovaskuläre Stents bezeichnete Gefäßstents zur Behandlung von Aneurysmen in Arterien implantiert. Unter einem Aneurysma versteht man eine Ausweitung oder Aussackung eines arteriellen Blutgefäßes infolge angeborener oder erworbener Wandveränderungen. Die Aussackung kann dabei die Gefäßwand als Ganzes erfassen oder es kann bei einem sog. falschen Aneurysma Blut aus dem Lumen des Gefäßes zwischen die Gefäßwandschichten treten und diese auseinander scheren. Die Nichtbehandlung eines Aneurysma kann im fortgeschrittenen Stadium zu einer Ruptur der Arterie führen, woraufhin der Patient innerlich verbluten kann.

Aneurysmen treten zwar häufig im Bereich der Bauchartierie (Aorta abdominalis) oder Brustarterie (Aorta thoracica) auf, ein Aneurysma kann aber auch im Bereich des aufsteigenden oder absteigenden Astes der Aorta (Aorta ascendens und Aorta descendens) auftreten. Der aufsteigende Ast der Aorta ist unmittelbar mit dem Herzen verbunden. Ausgehend von der Aortenwurzel (Sinus aortae) verläuft der aufsteigende Ast in leicht gekrümmter Form vom Herzen weg nach oben und geht dort in den Aortenbogen (Arcus aortae) über. Im Bereich des Aortenbogens zweigen die Kopfgefäße ab, u.a. die linke und die rechte Halsschlagader. Der Aortenbogen weist einen Kurvenverlauf von etwa 180° mit einem sehr engen Radius auf und verbindet den aufsteigenden Ast der Aorta mit dem absteigenden Ast.

Die zur Behandlung derartiger Aneurysmen verwendeten Stents bestehen aus einem hohlzylindrischen Metallrahmen, dessen Mantelfläche mit einer Textil- oder Polymerfolie abgedeckt ist, so dass sich ein hohlzylindrischer Körper ergibt. Zur Implantation wird der Stent radial zusammengedrückt, so dass sich seine Querschnittsfläche deutlich verringert. Der Stent wird dann mit Hilfe eines Einführsystems in den Bereich des Aneurysma gebracht, wo der Stent freigesetzt wird. Auf Grund der Federwirkung des Metallrahmens expandiert der Stent wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch den Stent und eine weitere Belastung der Aussackung wird verhindert.

Für die gewünschte Wirkung des Stents ist es nicht nur erforderlich, diesen axial so zu positionieren, dass er sich distal und proximal von dem Aneurysma in dem entsprechenden Blutgefäß verspannen kann, auch die radiale Ausrichtung des Stents ist häufig von entscheidender Bedeutung. Dies ist insbesondere dann der Fall, wenn proximal von dem Aneurysma weitere Gefäße von dem das Aneurysma aufweisenden Blutgefäß abzweigen, wie dies bspw. im Bereich des Aortenbogens der Falls ist, wo die arteriellen Kopfgefäße abzweigen. Damit die Versorgung dieser abzweigenden Gefäße nicht beeinträchtigt wird, sind die Stents häufig mit seitlichen Öffnungen versehen, durch die Blut aus dem Inneren des Stents austreten kann. Diese Öffnungen müssen nun im Bereich des Abganges der abzweigenden Gefäße positioniert werden, wozu es erforderlich ist, den Stent bei der Implantation nicht nur in seiner Längsrichtung zu verschieben, sondern auch um seine Längsrichtung zu drehen.

Zur Implantation werden diese Stents radial zusammengefaltet und dann mit Hilfe von endoluminal vorgeschobenen Kathetern in das Blutgefäß eingeführt und lagerichtig im Bereich des Aneurysma positioniert. Die richtige Lage des Stents kann dabei über Röntgenmarker kontrolliert werden, die auf dem Mantel des Stents, insbesondere im Bereich der Öffnungen für die Versorgung der abzweigenden Blutgefäße, vorgesehen sind.

Damit die Stents während der Positionierung im zusammengefalteten Zustand verbleiben, sind sie in einer Hülse oder einem Schlauch angeordnet, der den Stent radial nach innen drückt. Diese sog. Rückzughülle wird nach dem Positionieren des Stents im Bereich des Aneurysma zurückgezogen, wobei der Stent axial von einem Anschlagrohr gehalten wird, das auch als Pusher bezeichnet wird. Der Pusher liegt dabei in Anlage mit dem Stent und hält diesen in seiner axialen Lage, während die auch den Pusher umgebende Rückzughülle von dem Stent abgezogen wird, der sich dabei expandiert und in dem Blutgefäß verklemmt.

Die insoweit beschriebenen Einführsysteme arbeiten jedoch insbesondere in solchen Anwendungsfällen nicht zufriedenstellend, in denen der Stent in einem gekrümmten Abschnitt eines Blutgefäßes, bspw. im Aortenbogen, positioniert werden muss.

Die hier eingesetzten Stents weisen besonders große Abmaße auf, selbst im radial komprimierten, also zusammengefalteten Zustand haben sie noch einen Durchmesser von 6 bis 8 mm. Die hierfür verwendeten Rückzughüllen bestehen aus Polymerschläuchen, die meist aus Polyethylen oder Tetrafluorethylen hergestellt sind. Die Wandstärke dieser Polymerschläuche wird dabei so bemessen, dass sie dem Expansionsdruck des zusammengefalteten Stents standhält und auch über der Zeit stabil bleibt sowie keinem thermischen Kriechen unterliegt. Dies bedeutet jedoch, dass die Rückzughülle ein relativ hohes Flächenträgheitsmoment seines Querschnittprofils aufweist. Ferner sind die Rückzughüllen relativ steif, damit der Operateur nicht die Kontrolle über den Grad der Stentfreisetzung verliert.

Wenn nun derartige Einführsysteme, also mit radial zusammengefalteten Stents, die in einer steifen Rückzughülle platziert sind, bei engen Gefäßradien, wie im Aortenbogen, eingesetzt werden, neigen die Rückzughüllen wegen ihres großen Flächenträgheitsmoments zu Knickbildungen. Ein oder mehrere solche Knicke in der Rückzughülle verkanten sich dann beim Freisetzungsversuch im zusammengefalteten Stent und erschweren oder verhindern sogar eine vollständige Freisetzung im Gefäßbogen an der gewünschten Stelle.

Wenn derartige Einführsysteme verwendet werden, zieht der Operateur daher das Einführsystem nach der Positionierung in eine distale und damit geradere Gefäßregion zurück, um den Stent dort zu einem gewissen Grad freisetzen zu können. Daraufhin wird der zum Teil entfaltete Stent wieder in den Gefäßbogen eingeführt, was ein besonders riskantes Manöver ist, da die Gefahr der Gefäßwandperforation besteht. Wenn der Stent wieder richtig positioniert wurde, wird er dann mit großem Kraftaufwand ganz freigesetzt.

Neben der Gefahr der Gefäßwandperforation führt diese Handhabung häufig dazu, dass der Stent nicht mit der hinreichenden Präzision positioniert werden kann. Dazu trägt auch der hohe Kraftaufwand bei, der zur Überwindung der Knickstellen in der Rückzughülle aufgewandt werden muss.

Vor diesem Hintergrund liegt der vorliegenden Erfindung die Aufgabe zu Grunde, ein Einführsystem der eingangs genannten Art zu schaffen, bei dem die vorstehend genannten Nachteile vermieden werden. Insbesondere soll das neue Einführsystem eine zuverlässige Positionierung des Stents in dem Blutgefäß ermöglichen, ohne dass die Gefahr besteht, dass die Gefäßwand zusätzlich beschädigt wird.

Die der Erfindung zu Grunde liegende Aufgabe wird durch das Einführsystem gemäß Anspruch 1 vollkommen gelöst.

Die Erfinder der vorliegenden Anmeldung haben nämlich erkannt, dass es möglich ist, die aus dem Stand der Technik bekannte Rückzughülle sozusagen zweiteilig auszuführen, wobei der vordere Abschnitt, der den Stent umgibt und in seiner komprimierten Lage hält, hochflexibel ausgebildet ist, so dass er sich den Krümmungen des Blutgefäßes leicht anpassen kann. Obwohl auch dabei Knickstellen in dem schlauchförmigen vorderen Abschnitt entstehen können, behindern diese das Zurückziehen des vorderen Abschnittes nicht, da dieser aus hochflexiblem Material besteht. Dieses Material kann durch die Knickstellen und Falten sozusagen hindurchgleiten, wozu kein großer Kraftaufwand erforderlich ist. Die gleichen Vorteile ergeben sich beim Positionieren in dem Blutgefäß, denn bei einer Rotation oder Biegung des hochflexiblen vorderen Abschnittes durch die Bewegungen mit dem Einführsystem rollen die entstehenden Falten oder Knicke sehr leicht auf dem Umfang ab und erleichtern so das Positionieren des Stents.

Der hintere Abschnitt dagegen ist aus einem deutlich steiferen Material gefertigt, so dass er die erforderlichen Dreh- und Zugkräfte über die vielen Dezimeter seiner Länge von dem außerhalb des Körpers verbleibenden Betätigungsbereich bis zu dem vorderen Abschnitt der Rückzughülle übertragen kann. Eine solche zweiteilige Vorrichtung ist zum Beispiel aus der EP-A-0941716 bekannt.

Dabei ist es bevorzugt, wenn der vordere und der hintere Abschnitt miteinander verklebt sind.

Bei dieser Maßnahme ist von Vorteil, dass eine sichere flächige Verbindung zwischen dem vorderen und dem hinteren Abschnitt hergestellt wird, über die die entsprechenden Kräfte Übertragen werden können.

Gemäß der Erfindung weist der vordere Abschnitt einen Textilschlauch, also einen vorzugsweise gewebten Schlauch mit Textilstruktur auf, der vorzugsweise aus einem nahtlos gewebten Textilmaterial, weiter vorzugsweise aus Polyester-Material (PET) gefertigt ist.

Bei dieser Maßnahme ist von Vorteil, dass für den vorderen Abschnitt der Rückzughülle ein vorzugsweise nahtlos gewebter Textilschlauch eingesetzt wird, der auf Grund seiner Textil-, vorzugsweise Webstruktur den Stent mit großer Umfangskraft radial zusammengedrückt halten kann. Die Textilstruktur sorgt gleichzeitig für eine geringe Elastizität und eine hohe Festigkeit in axialer Zugrichtung, was für das sichere Positionieren des Stents von entscheidendem Vorteil ist. Ferner ist das Flächenträgheitsmoment der Summe aller Längsfasern im Querschnitt des Textilschlauchs sehr gering, so dass sich die hochflexiblen Eigenschaften des vorderen Abschnittes der Rückzughülle ergeben, die erfindungsgemäß ein leichtes Positionieren sowie Freisetzen des Stents ermöglichen.

Zwar treten bei einem derartigen Textilschlauch ebenfalls Falten und Knickstellen auf, bei einer Rotation oder Biegung des Textilschlauchs durch die zum Positionieren des Stents erforderlichen Bewegungen mit dem Einführsystem rollen diese Falten jedoch sozusagen sehr leicht auf dem Umfang ab, so dass die Positionierung nicht behindert wird. Gleiches gilt beim Abziehen des Textilschlauches von dem Stent nach dessen Positionierung in dem Gefäßbogen, da das textile Material sozusagen durch die Falten und Knicke hindurchgleitet. Der erforderliche Kraftaufwand zum Freisetzen des Stents reduziert sich also auf die Überwindung der Reibung zwischen Stent und Textilschlauch. Ein Verkanten des Stents an den Falten ist somit nicht möglich und der Stent kann sicher in einem engen Gefäßbogen abgesetzt werden.

Allgemein ist es bevorzugt, wenn der hintere Abschnitt einen Zugschlauch aufweist, der vorzugsweise aus Polymermaterial, insbesondere aus Polyethylen-Material (PE), vorzugsweise aus hochdichtem PE (HD-PE) gefertigt ist.

Hier ist von Vorteil, dass der hintere Abschnitt der Rückzughülle durch einen relativ steifen Zugschlauch gebildet wird, der zur Übertragung von Dreh- und Zugkräften auf den Textilschlauch geeignet ist. Auf diese Weise wird ein sicheres Einführen und Positionieren des Stents ermöglicht, obwohl der vordere Abschnitt aus einem hochflexiblen Material gefertigt ist.

Dabei ist es bevorzugt, wenn in dem hinteren Abschnitt ein Anschlagrohr angeordnet ist, das am Stent axial anliegt.

Bei dieser Maßnahme ist von Vorteil, dass das Anschlagrohr zum einen zum Vorschieben des Stents verwendet werden kann, so dass die Schubkraft nicht lediglich über den hinteren Abschnitt, also den Zugschlauch, ausgeübt werden muss. Das Anschlagrohr drückt beim Vorschieben sozusagen auf das distale Ende des Stents, der zusammen mit dem fest darauf sitzenden vorderen Abschnitt so in dem Blutgefäß vorgeschoben werden kann. Zur Rotation des Stents wird der Zugschlauch verdreht, der dabei den vorderen Abschnitt und somit auch den Stent mitnimmt.

Mit anderen Worten, für den Vorschub des Stents in das Blutgefäß hinein ist im Wesentlichen das Anschlagrohr, darüber hinaus aber auch der Zugschlauch vorgesehen, während für das Zurückziehen aus dem Blutgefäß sowie die Rotation des Stents der Zugschlauch zusammen mit dem Textilschlauch vorgesehen sind. Eine weitere Aufgabe des Anschlagrohres besteht darin, den einmal positionierten Stent an Ort und Stelle zu halten, während mit Hilfe des Zugschlauches der Textilschlauch in distaler Richtung von dem Stent abgezogen wird.

Allgemein ist es bevorzugt, wenn der vordere Abschnitt am proximalen Ende lösbar mit einem kegelförmigen Spitzenteil verbunden ist, der vorzugsweise eine weiche kegelförmige Spitze aus einem weichen Material wie bspw. Weich-Polyurethan (Weich-PU) aufweist, die unverlierbar auf einem kegelförmigen Spitzenflansch aus einem harten Material wie bspw. Hart-PU sitzt.

Bei dieser Maßnahme ist von Vorteil, dass das vordere, also proximale Ende des vorderen Abschnittes, also insbesondere des Textilschlauches, mit einer kegelförmigen Spitze verbunden ist, die einerseits eine hinreichende Steifigkeit aufweist, um in einem Blutgefäß vorgeschoben zu werden, andererseits aber am proximalen Ende hinreichend weich ist, um Verletzungen des Blutgefäßes zu verhindern.

Insbesondere im Zusammenhang mit diesem kegelförmigen Spitzenteil ist der Einsatz eines Textilschlauches als vorderer Abschnitt der Rückzughülle von Vorteil, denn trotz der hochflexiblen Eigenschaften des Textilschlauches lässt sich der Stent sicher in dem Blutgefäß positionieren.

Dabei ist es bevorzugt, wenn der vordere Abschnitt vorzugsweise thermisch auf den Spitzenflansch aufgeschrumpft ist, wobei der Spitzenflansch weiter vorzugsweise an seinem distalen Ende eine umlaufende Wulst aufweist, auf die der vordere Abschnitt aufgeschrumpft ist. Weiter ist es bevorzugt, wenn der vordere Abschnitt mit seiner vorderen Kante in einer an dem Spitzenflansch vorgesehenen, umlaufenden Vertiefung liegt.

Hier ist von Vorteil, dass das proximale Ende des Textilschlauches während des Vorschiebens nicht unbeabsichtigt abgleiten und damit das Endothel verletzen kann.

Besonders von Vorteil ist es dabei, wenn der Textilschlauch durch thermisches Aufschrumpfen an dem Spitzenflansch fixiert wird. Dieses Verfahren nutzt nämlich die Tatsache aus, dass Textilien bei thermischer Überbeanspruchung "einlaufen", wobei über den vorzugsweise abgerundeten Wulst des Spitzenflansches ein Formschluss zum Textilschlauch entsteht, der so bemessen ist, dass er den Reibungskräften beim Einführen widersteht. Beim Rückzug des Textilschlauches kann diese formschlüssige Verbindung jedoch leicht überwunden werden.

Wenn die vordere Kante des Textilschlauches dabei in einer an dem Spitzenflansch vorgesehenen, umlaufenden Vertiefung liegt, kommt diese vordere Kante nicht in Kontakt mit der Gefäßinnenwand. Hierdurch wird zum einen das Endothel geschützt, zum anderen aber ein Abrollen des Textilschlauches von dem Spitzenflansch verhindert.

Allgemein ist es noch bevorzugt, wenn ein Drahtführungskatheter vorgesehen ist, der mittig durch den Spitzenteil sowie den Stent und den hinteren Abschnitt verläuft und mit dem Spitzenteil auf Zugbelastung verbunden ist.

Dieser Drahtführungskatheter dient zum einen dazu, das Einführsystem über einen Führungsdraht sicher in dem Blutgefäß verschieben zu können. Zum anderen dient der Drahtführungskatheter dazu, nach dem Freisetzen des Stents den Spitzenteil durch den jetzt expandierten Stent aus dem Blutgefäß zurückzuziehen.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in Bezug auf diese nachstehend näher beschrieben. Es zeigen:
- Fig. 1: eine schematische Darstellung eines im Bereich des Aortenbogens implantierten endovaskulären Stents;
- Fig. 2: ein Einführsystem, um den Stent aus Fig. 1 in dem Aortenbogen zu platzieren und dort freizusetzen;
- Fig. 3: in einer schematischen und ausschnittsweisen Seitenansicht das im Aortenbogen positionierte Einführsystem aus Fig. 2;
- Fig. 4: eine Darstellung wie Fig. 3, jedoch mit von dem Spitzenflansch des Einführsystems abgezogenem Textilschlauch;
- Fig. 5: eine Darstellung wie Fig. 4, jedoch mit weiter zurückgezogenem Textilschlauch; und
- Fig. 6: eine Darstellung wie Fig. 5, jedoch mit vollständig freigesetztem Stent, jedoch mit noch proximal von dem Stent liegendem Spitzenteil.

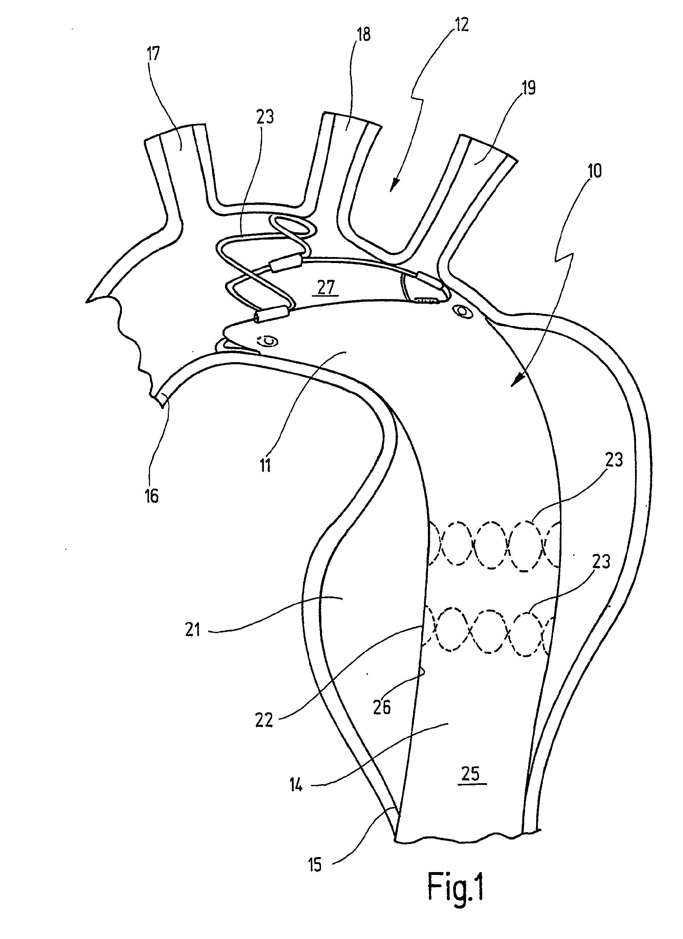

In Fig. 1 ist mit 10 ein Stent gezeigt, der mit seinem proximalen Ende 11 in dem Aortenbogen 12 und mit seinem distalen Ende 14 in der Aorta descendens 15 verankert ist.

In Fig. 1 ist ebenfalls schematisch das Aortensystem dargestellt, das jetzt zunächst kurz erläutert werden soll.

Der aufsteigende Ast 16 (A. ascendens) der Aorta ist über die in Fig. 1 nicht gezeigte Aortenwurzel (Sinus aortae) mit der ebenfalls nicht dargestellten linken Kammer des Herzens verbunden. Die Aorta ascendens 16 ist über den Aortenbogen 12 mit der Aorta descendens 15 verbunden. Im Bereich des Aortenbogens 12 gehen arterielle Kopfgefäße ab, nämlich der Arterienstamm Truncus brachiocephalicus 17, die Arteria carotis communis 18 und die Arteria subclavia sinistra 19.

In der Aorta descendens 15 ist bei 21 ein Aneurysma dargestellt, das durch den Stent 10 sozusagen überbrückt ist. Der aus der Aorta ascendens 16 kommende Blutstrom gelangt über den Aortenbogen 12 in das proximale Ende 11 des Stents 10 und verlässt diesen am distalen Ende 14. Zu diesem Zweck weist der Stent 10 einen hohlzylindrischen Körper 22 auf, der durch in Fig. 1 schematisch angedeutete Ringe 23 aus mäanderförmigen Metallstützen gebildet ist, die durch Prothesenmaterial 25 miteinander verbunden sind. Das Prothesenmaterial 25 ist in bekannter Weise ein textiles Material oder eine Folie und ist durch Nähen, Kleben oder Einschmelzen an den Ringen 23 befestigt.

Auf diese Weise wird der Durchgang durch den Stent 10 offen gehalten, so dass sich der hohlzylindrische Körper 22 mit seinem bei 26 angedeuteten Mantel bildet.

An seinem proximalen Ende 11 ist der Stent 10 zu den arteriellen Kopfgefäßen 17, 18 und 19 hin mit einer V-förmigen Öffnung 27 versehen, durch die hindurch insbesondere die Kopfgefäße 18 und 19 mit Blut versorgt werden.

Der Stent 10 ist selbstexpandierend, d.h. er verankert sich durch radialen Druck nach außen an seinem proximalen Ende 11 sowie an seinem distalen Ende 14 in dem Aortenbogen 12 bzw. in der Aorta descendens 15.

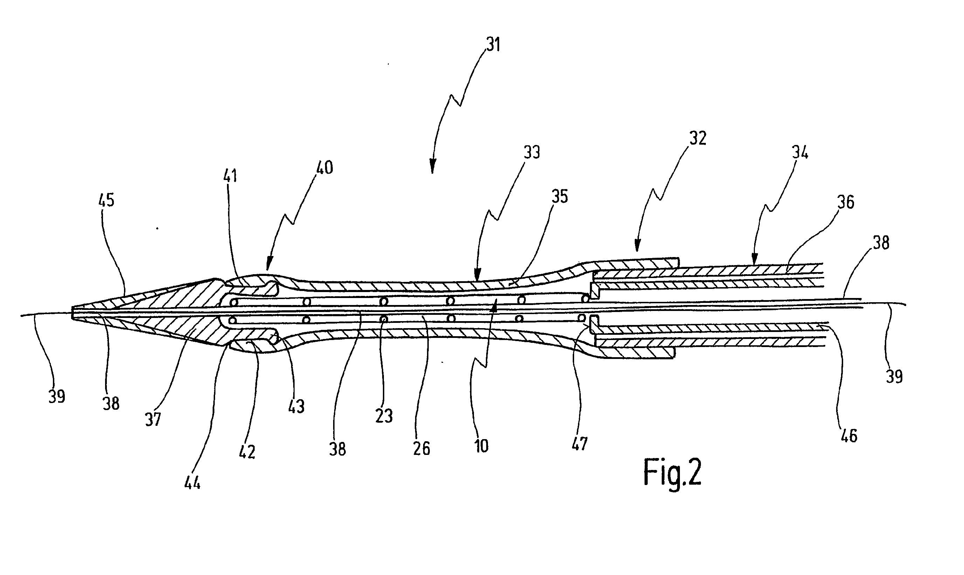

Die Positionierung und Freisetzung des Stents 10 aus Fig. 1 erfolgt über ein in Fig. 2 bei 31 angedeutetes Einführsystem, das in Fig. 2 in schematischer Seitenansicht und nicht maßstabsgetreu gezeigt ist.

Der Stent 10 ist in Fig. 2 radial stark komprimiert, schematisch sind die Ringe 23 sowie der Mantel 26 gezeigt. Der Stent 10 befindet sich in einer Rückzughülle 32, die einen vorderen Abschnitt 33 und einen hinteren Abschnitt 34 aufweist. Der vordere Abschnitt 33, der den Stent 10 aufnimmt und radial komprimiert, ist ein Textilschlauch 35 aus einem textilen Material wie bspw. Polyester (PET). Dieses textile Material ist wegen der gewebten Textilstruktur hochflexibel, so dass das Flächenträgheitsmoment der Summe aller Längsfasern im Querschnitt des Textilschlauches 35 sehr gering ist.

Der hintere Abschnitt 34 der Rückzughülle 32 ist ein Zugschlauch 36 aus einem Polymermaterial, wie bspw. hochdichtes Polyethylen (HD-PE). Der Zugschlauch 36 ist sehr viel steifer als der Textilschlauch 35, so dass er Zug- und Drehkräfte auf den Textilschlauch 35 und über diesen auf den Stent 10 übertragen kann. Zu diesem Zweck sind der Textilschlauch 35 und der Zugschlauch 36 an ihrer überlappenden Nahtstelle miteinander verklebt.

Im Bereich des proximalen Endes 11 des Stents 10 ist ein kegelförmiger Spitzenflansch 37 aus einem harten Material wie bspw. Hart-PU (Polyurethan) angeordnet. Dieser Spitzenflansch 37 wird zentrisch von einem Drahtführungskatheter 38 durchsetzt, der auch durch den Stent 10 sowie den hinteren Abschnitt 34 der Zughülle 32, also den Zugschlauch 36, verläuft. Durch den Drahtführungskatheter 38 verläuft ein bei 39 angedeuteter Führungsdraht, über den das Einführsystem 31 in einem Blutgefäß geführt wird, wenn es in dieses hineingeschoben wird.

Der Spitzenflansch 37 weist an seinem distalen Ende 40 einen ringförmigen Ansatz 41 auf, der eine umlaufende Vertiefung 42 sowie distal dazu eine umlaufende, abgerundete Wulst 43 aufweist.

Der Textilschlauch 35 ist mit seiner vorderen Kante 44 über die umlaufende Wulst 43 geschoben, so dass die vordere Kante 44 in der Vertiefung 42 liegt. Durch Erhitzen ist der Textilschlauch auf das distale Ende 40 aufgeschrumpft, so dass der Textilschlauch 35 beim Einschieben des Einführsystems 31 in ein Blutgefäß nicht von dem kegelförmigen Spitzenflansch 37 heruntergeschoben werden kann.

Auf dem harten, kegelförmigen Spitzenflansch 37 sitzt unverlierbar eine weiche Spitze 45 aus Weich-PU, um zu verhindern, dass beim Vorschieben des so gebildeten Spitzenteils aus Spitze 45 und Spitzenflansch 37 das Blutgefäß innen verletzt wird. Der Drahtführungskatheter 38 verläuft auch durch die Spitze 45.

In dem hinteren Abschnitt 34, also in dem Zugschlauch 36, ist noch ein Anschlagrohr 46 vorgesehen, das mit seiner Stirnseite 47 axial an den Stent 10 anstößt. Der Stent 10 wiederum liegt mit seinem proximalen Ende 11 in dem ringförmigen Ansatz 41 und stößt somit axial gegen den kegelförmigen Spitzenflansch 37.

Zum Einführen des Stents 10 in ein Blutgefäß wird - wie allgemein bekannt - zunächst der Führungsdraht 39 gelegt, auf den dann das Einführsystem 31 aufgeschoben wird. Beim Vorschieben des Einführsystems 31 drückt das Anschlagrohr 46 über seine Stirnseite 47 auf den Stent 10, der mit seinem proximalen Ende 11 auf den kegelförmigen Spitzenflansch 37 drückt, so dass dieser und die auf ihm sitzende Spitze 45 vorgeschoben werden. Der Textilschlauch 35 drückt dabei den Stent 10 radial nach innen zusammen, wobei er gleichzeitig eine glatte Oberfläche darbietet, so dass das Einführsystem in dem Blutgefäß leicht verschoben werden kann, ohne dass die Gefahr der Beschädigung des Endothels besteht.

Da der Textilschlauch 35 wie auch der Stent 10 selbst hochflexibel ist, passt sich das Einführsystem zwischen dem Spitzenflansch 37 und dem hinteren Abschnitt 34 der Rückzughülle 32 auch engen Radien von Blutgefäßen an, wobei eine entsprechende Vorschubkraft dennoch ausgeübt werden kann, da der Stent 10, der die Kraft von dem Schubrohr 46 auf den Spitzenflansch 37 überträgt, wegen des Textilschlauches 35 nicht seitlich "ausbrechen" kann.

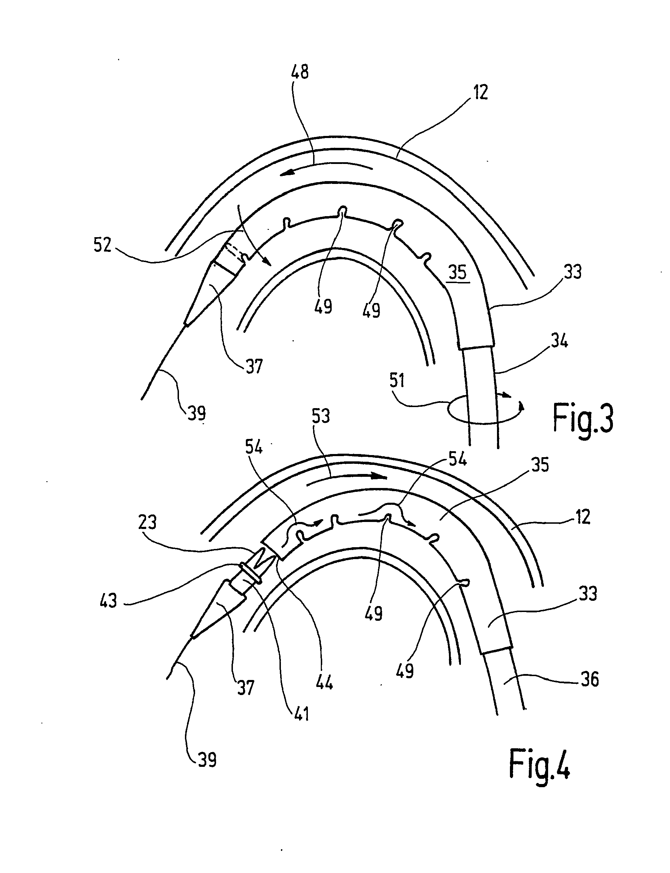

Der Zustand des Einschiebens des Einführsystems 31 in den Aortenbogen 12 ist in Fig. 3 schematisch dargestellt. Mit 48 ist dort die Einführrichtung bezeichnet, in der das Einführsystem 31 in den Aortenbogen 12 hineingeschoben wird.

Damit der Stent 10 die richtige radiale Lage in dem Aortenbogen 12 einnimmt, kann über den hinteren Abschnitt 34 eine bei 51 angedeutete Drehbewegung ausgeübt werden, die über den Textilschlauch 35 auf den Spitzenflansch 37 und den Stent 10 übertragen wird. Beim Vorschieben in Einführrichtung 48 führt das Einführsystem 31 an seinem vorderen Ende auch eine bei 52 gezeigte Biegebewegung durch, durch die Falten und Knicke 49 in dem Textilschlauch 35 entstehen können.

Wenn der Stent 10 axial und radial richtig positioniert wurde, wird der Zugschlauch 36 in Richtung einer in Fig. 4 gezeigten Abzugbewegung 53 zurückgezogen. Das Anschlagrohr 46 bleibt dabei mit seiner Stirnseite 47 in Anlage mit dem Stent 10, so dass dieser seine axiale Lage nicht verändert.

Beim Abziehen des Textilschlauches 35 gleitet diese zunächst mit ihrer vorderen Kante 44 über den Wulst 43, so dass der Textilschlauch 35 von dem Spitzenflansch 37 freikommt. Die hierfür erforderliche Kraft kann über den Zugschlauch 36 auf den Textilschlauch 35 deshalb ausgeübt werden, weil deren Elastizität in Längsrichtung sehr gering ist und ferner über das Anschlagrohr 46 und den Stent 10 eine entsprechende Gegenkraft auf den Spitzenflansch 37 ausgeübt werden kann.

In Fig. 4 ist die Situation gezeigt, bei der der Textilschlauch 35 gerade von dem ringförmigen Ansatz 41 des Spitzenflansches 37 freigekommen ist. Ein vorderer Ring 23 des Stents 10 liegt jedoch noch in dem ringförmigen Ansatz 41, wie es in Fig. 4 zu erkennen ist.

Der Textilschlauch 35 gleitet jetzt beim weiteren Zurückziehen in Abzugsrichtung 53 sozusagen durch die Falte 49 hindurch, was durch Pfeile 54 angedeutet ist. Ein Verkanten des Textilschlauches 35 an dem Stent 10 ist somit ausgeschlossen.

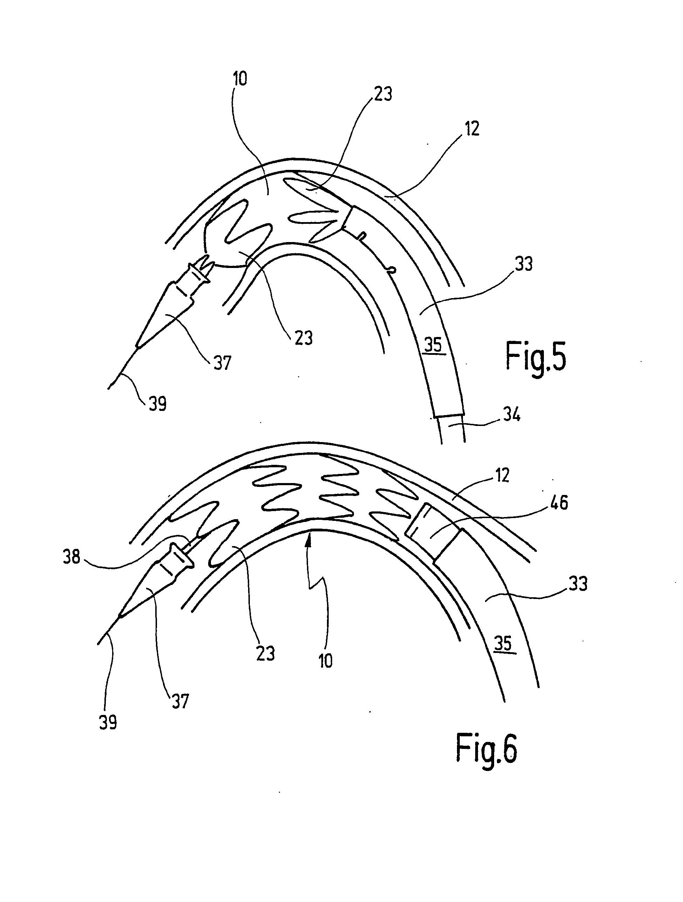

Beim weiteren Zurückziehen des Zugschlauches 36 und damit des Textilschlauches 35 wird der Stent 10 immer mehr freigegeben, wie es in Fig. 5 zu sehen ist, wo bereits ein zweiter Ring 23 aus dem Textilschlauch 35 freigekommen ist.

In Fig. 6 schließlich ist der gesamte Stent 10 aus dem Textilschlauch 35 freigekommen, der vordere Abschnitt 33 wurde sogar bereits teilweise von dem Anschlagrohr 46 abgezogen.

Auch der vordere Ring 23 des Stents 10 ist nun aus dem Spitzenflansch 37 freigekommen, so dass dieser und die Spitze 45 mit Hilfe des Drahtführungskatheters 38 jetzt durch den Stent 10 hindurch in das Anschlagrohr 46 gezogen werden können, bevor das restliche Einführsystem aus dem Blutgefäß entfernt wird.

## Patentansprüche

1. Einführsystem mit einem selbstexpandierenden Stent (10) zur Implantation in ein Blutgefäß (15), insbesondere im Bereich des Aortenbogens (12), wobei der Stent (10) einen hohlzylindrischen Körper (22) aufweist, der zur Implantation radial komprimiert ist, und mit einer den Stent (10) umgebenden und diesen radial zusammendrückenden Rückzughülle (32) zur Positionierung und Freisetzung des Stents (10) in dem Blutgefäß (15),
**dadurch gekennzeichnet, dass** die Rückzughülle (32) einen hochflexiblen vorderen Abschnitt (33), der den Stent (10) umgibt und in seinem komprimierten Zustand hält, sowie einen mit dem vorderen Abschnitt (33) verbundenen, steiferen hinteren Abschnitt (34) aufweist, der zur Übertragung von Dreh- und Zugkräften auf den vorderen Abschnitt (33) ausgelegt ist, wobei der vordere und der hintere Abschnitt (33, 34) vorzugsweise aus verschiedenen Materialien gefertigt sind, und wobei der vordere Abschnitt (33) einen gewebten Textilschlauch (35) mit Textilstruktur darstellt.

2. Einführsystem nach Anspruch 1, **dadurch gekennzeichnet, dass** der vordere und der hintere Abschnitt (33, 34) miteinander verklebt sind.

3. Einführsystem nach Anspruch 2, **dadurch gekennzeichnet, dass** der Textilschlauch (35) aus einem nahtlos gewebten Textilmaterial gefertigt ist.

4. Einführsystem nach Anspruch 3, **dadurch gekennzeichnet, dass** der Textilschlauch (35) aus Polyester-Material (PET) gefertigt ist.

5. Einführsystem nach einem der Ansprüche 1 bis 4, **dadurch gekennzeichnet, dass** der hintere Abschnitt (34) einen Zugschlauch (36) aufweist, der vorzugsweise aus Polymermaterial, insbesondere aus Polyethylen-Material gefertigt ist.

6. Einführsystem nach einem der Ansprüche 1 bis 5, **dadurch gekennzeichnet, dass** der vordere Abschnitt (33) am proximalen Ende (11) lösbar mit einem kegelförmigen Spitzenteil (37, 45) verbunden ist, der eine kegelförmige weiche Spitze (45) vorzugsweise aus einem weichem Material wie Weich-Polyurethan aufweist, die unverlierbar auf einem harten Spitzenflansch (37) aus einem harten Material wie Hart-Polyurethan sitzt.

7. Einführsystem nach Anspruch 6, **dadurch gekennzeichnet, dass** der vordere Abschnitt (33) vorzugsweise thermisch auf den Spitzenflansch (37) aufgeschrumpft ist.

8. Einführsystem nach Anspruch 6 oder 7, **dadurch gekennzeichnet, dass** der Spitzenflansch (37) an seinem distalen Ende (40) eine umlaufende Wulst (43) aufweist, auf die der vordere Abschnitt (33) aufgeschrumpft ist.

9. Einführsystem nach einem der Ansprüche 6 bis 8, **dadurch gekennzeichnet, dass** der vordere Abschnitt (33) mit seiner vorderen Kante (44) in einer an dem Spitzenflansch (37) vorgesehenen, umlaufenden Vertiefung (42) liegt.

10. Einführsystem nach einem der Ansprüche 1 bis 9, **dadurch gekennzeichnet, dass** in dem hinteren Abschnitt (34) ein Anschlagrohr (46) angeordnet ist, das am Stent (10) axial anliegt.

11. Einführsystem nach einem der Ansprüche 7 bis 10, **dadurch gekennzeichnet, dass** ein Drahtführungskatheter (38) vorgesehen ist, der mittig durch den Spitzenteil (37, 45) sowie den Stent (10) und den hinteren Abschnitt (34) verläuft und mit dem Spitzenflansch (37) auf Zugbelastung verbunden ist.

## Claims

1. Delivery system with a self-expanding stent (10) for implantation into a blood vessel (15), in particular in the region of the aortic arch (12), said stent (10) comprising a hollow cylindrical body (22) which is radially compressed for implantation, and with a pull-back sleeve (32) which surrounds and radially compresses the stent (10) for positioning and releasing in the blood vessel (15), **characterized in that** the pull-back sleeve (32) comprises a highly flexible front section (33) which surrounds the stent (10) and which maintains said stent (10) in its compressed state, and a more rigid rear section (34) which is connected to the front section (33) and which is designed to transmit rotational and traction forces to the front section (33), the front and rear sections (33, 34) preferably being made from different materials, and wherein the front section (33) comprises a woven textile tube (35) with textile structure.

2. The delivery system as claimed in claim 1, **characterized in that** the front and rear sections (33, 34) are adhesively bonded to one another.

3. The delivery system as claimed in claim 2, **characterized in that** the textile tube (35) is made from a seamlessly woven textile material.

4. The delivery system as claimed in claim 3, **characterized in that** the textile tube (35) is made from polyester material (PET).

5. The delivery system as claimed in one of claims 1 to 4, **characterized in that** the rear section (34) comprises a traction tube (36) which is preferably made from polymer material, in particular from polyethylene material.

6. The delivery system as claimed in one of claims 1 through 5, **characterized in that** the front section (33) at the proximal end (11) is connected in a detachable manner to a conical tip part (37, 45) having a conical soft tip (45) preferably made from a soft material such as soft polyurethane, which soft tip (45) sits nonreleasably on a hard tip flange (37) made from a hard material such as hard polyurethane.

7. The delivery system as claimed in claim 6, **characterized in that** the front section (33) is preferably shrunk thermally onto the tip flange (37).

8. The delivery system as claimed in claim 6 or 7, **characterized in that** the tip flange (37) has, at its distal end (40), a circumferential bead (43) onto which the front section (33) is shrunk.

9. The delivery system as claimed in one of claims 6 to 8, **characterized in that** the front section (33) lies with its front edge (44) in a circumferential depression (42) provided on the tip flange (37).

10. The delivery system as claimed in one of claims 1 through 9, **characterized in that** an abutment bushing (46) is arranged in the rear section (34) and bears axially on the stent (10).

11. The delivery system as claimed in one of claims 7 through 10, **characterized in that** a guide wire catheter (38) is provided which extends centrally through the tip part (37, 45) and the stent (10) and the rear section (34) and is connected to the tip flange (37) under tensile loading.

## Revendications

1. Système d'introduction avec un stent autoexpansible (10) destiné à être implanté dans un vaisseau sanguin (15), notamment au niveau de la crosse de l'aorte (12), le stent (10) présentant un corps cylindrique creux (22) comprimé radialement en vue de son implantation, et avec une gaine apte à être retirée (32) entourant ledit stent (10) et le comprimant radialement en vue de son positionnement et de sa libération dans le vaisseau sanguin (15),
**caractérisé en ce que** la gaine apte à être retirée (32) présente une partie avant (33) très flexible entourant le stent (10) et le maintenant à l'état comprimé, ainsi qu'une partie arrière (34) plus rigide, assemblée avec ladite partie avant (33) et conçue pour transmettre des efforts de torsion et de traction à ladite partie avant (33), les parties avant et arrière (33, 34) étant fabriquées de préférence à partir de matériaux différents et la partie avant (33) présentant une gaine textile (35) tissée à structure textile.

2. Système d'introduction selon la revendication 1, **caractérisé en ce que** la partie avant et la partie arrière (33, 34) sont assemblées par collage.

3. Système d'introduction selon la revendication 2, **caractérisé en ce que** la gaine textile (35) est en matériau textile tissé sans couture.

4. Système d'introduction selon la revendication 3, **caractérisé en ce que** la gaine textile (35) est en polyester (PET).

5. Système d'introduction selon l'une quelconque des revendications 1 à 4, **caractérisé en ce que** la partie arrière (34) présente un flexible de traction (36), de préférence en matériau polymère, notamment en polyéthylène.

6. Système d'introduction selon l'une quelconque des revendications 1 à 5, **caractérisé en ce que**, au niveau de son extrémité proximale (11), la partie avant (33) est assemblée de manière amovible avec une pièce pointue (37, 45) de forme conique qui présente une pointe souple (45) de forme conique, de préférence en matériau souple tel que du polyuréthane souple, montée imperdable sur un corps pointu (37) dur en matériau dur tel que du polyuréthane rigide.

7. Système d'introduction selon la revendication 6, **caractérisé en ce que** la partie avant (33) est rétractée, de préférence thermorétractée sur le corps pointu (37).

8. Système d'introduction selon la revendication 6 ou la revendication 7, **caractérisé en ce que** le corps pointu (37) présente, à son extrémité distale (40), un bourrelet périphérique (43), sur lequel la partie avant (33) est rétractée.

9. Système d'introduction selon l'une quelconque des revendications 6 à 8, **caractérisé en ce que** l'arête avant (44) de la partie avant (33) est logée dans une gorge périphérique (42) ménagée sur le corps pointu (37).

10. Système d'introduction selon l'une quelconque des revendications 1 à 9, **caractérisé en ce qu'**un tube de butée (46), qui coopère en appui axial avec le stent (10), est agencé dans la partie arrière (34).

11. Système d'introduction selon l'une quelconque des revendications 7 à 10, **caractérisé en ce qu'**il est prévu un cathéter guide-fil (38) qui traverse en leur centre la pièce pointue (37, 45), le stent (10) et la partie arrière (34) et qui est assemblé avec le corps pointu (37) de façon que ces éléments se déplacent solidairement en cas de sollicitation en traction.
